# DEMANDE DE BREVET EUROPEEN

(11) **EP 3 556 425 A1**
(43) Date de publication de la demande: **23.10.2019**
(21) Numéro de dépôt: 19169965.1
(22) Date de dépôt: 17.04.2019
(51) Int. Cl.: A61M 37/00

(54) **DISPOSITIF D'INJECTION D'IMPLANT DOTÉ DE MOYENS DE RETENUE PAR FRICTION**

(30) Priorité: 18.04.2018 FR 1853404
(71) Demandeur: NEMERA LA VERPILLIERE, 38290 La Verpillière (FR)
(72) Inventeur: PAINCHAUD, Gaëtan, 69340 FRANCHEVILLE (FR); DUGAND, Pascal, 38780 ESTRABLIN (FR); MEGARD, Thomas, 71960 LA ROCHE-VINEUSE (FR)
(74) Mandataire: LLR

(57) **Abrégé**

L'invention concerne un dispositif d'injection d'implant (10), comportant :
- une aiguille d'injection (22),
- un corps de réception (24) d'au moins un implant (26, 28),
- des moyens d'injection comprenant :
. une tige de poussée (52), disposée en amont de l'implant (26, 28) logé dans le corps de réception (24) et configurée pour pousser l'implant (26, 28) au travers de l'aiguille d'injection (22) entre une position initiale et une position finale,
. des moyens de poussée sur la tige de poussée (52) entre la position initiale et la position finale,
. des moyens de retenue par friction de la tige de poussée (52) par rapport à l'aiguille d'injection (22), s'opposant au déplacement de la tige de poussée (52) vers sa position finale, les moyens de retenue étant actionnables par un utilisateur.

## Description

L'invention concerne le domaine technique de l'injection d'un ou plusieurs implants dans le corps d'un patient.

On connaît des dispositifs d'injection d'implant, comprenant une aiguille creuse fixée à un logement recevant un implant. L'implant est injecté au moyen d'une tige de poussée, laquelle vient pousser l'implant à travers l'aiguille creuse puis au-delà pour permettre l'injection de l'implant dans le corps d'un patient.

On connaît en particulier du document US20090281520A1 un dispositif d'injection d'implant dans lequel l'injection de l'implant peut se faire par appui glissant sur un bouton, le bouton étant alors entraîné par l'utilisateur dans une direction sensiblement parallèle à la direction d'injection. Ainsi, avant actionnement, la tige de poussée est rétractée et le bouton se trouve du côté de l'extrémité proximale du dispositif, c'est-à-dire l'extrémité opposée à l'extrémité d'injection. Lors de l'appui glissant sur le bouton par l'utilisateur, le bouton glisse dans une direction sensiblement parallèle à la direction d'injection et entraîne la tige de poussée, laquelle pousse l'implant et permet son injection.

Toutefois, un tel actionnement par appui glissant ne permet pas aisément à l'utilisateur d'injecter des implants de grande longueur ou bien plusieurs implants. En effet, cet actionnement est généralement réalisé par le pouce de l'utilisateur, lequel possède une course réduite pour l'actionnement. De plus un tel actionnement ne permet pas de maintenir une grande précision d'injection sur une grande course. En effet, le risque de mouvement non souhaité dans une direction sensiblement parallèle à la direction d'injection est plus élevé lorsque la longueur de l'actionnement augmente, ce qui n'est pas souhaitable pour garantir une injection correcte, notamment concernant la profondeur d'injection dans le corps du patient, et pour éviter une rupture de l'implant ou une blessure du patient.

Ainsi, compte tenu de leur type d'actionnement, ces dispositifs d'injection d'implant ne permettent pas d'injecter aisément un implant de grande longueur ou bien une pluralité d'implants.

La présente invention vise notamment à fournir un dispositif d'injection d'implant, lequel permet d'injecter aisément un implant de grande longueur et/ou une pluralité d'implants.

A cet effet, l'invention a notamment pour objet un dispositif d'injection d'implant, comportant :
- une aiguille d'injection,
- un corps de réception d'au moins un implant,
- des moyens d'injection comprenant :
   - une tige de poussée, disposée en amont de l'implant logé dans le corps de réception et configurée pour pousser l'implant au travers de l'aiguille d'injection entre une position initiale et une position finale,
   - des moyens de poussée sur la tige de poussée entre la position initiale et la position finale,
   - des moyens de retenue par friction de la tige de poussée par rapport à l'aiguille d'injection, s'opposant au déplacement de la tige de poussée vers sa position finale, les moyens de retenue étant actionnables par un utilisateur.

Ainsi, on propose de réaliser l'injection en utilisant des moyens de poussée permettant de faciliter le déplacement de la tige de poussée, et en faisant en outre régler par l'utilisateur la retenue de la tige de poussée, plutôt qu'en demandant à l'utilisateur d'actionner la tige de poussée sur une longueur relativement longue. En d'autres termes, en combinant l'utilisation de moyens de poussée et de moyens de retenue par friction, le mouvement à effectuer par l'utilisateur pour permettre l'injection de l'implant est en particulier de faible amplitude, ce qui permet d'augmenter la taille et/ou le nombre des implants que l'on peut injecter une fois l'aiguille insérée dans le corps du patient. En d'autres termes, l'utilisateur commande facilement, et à la demande, la poussée exercée sur la tige de poussée via les moyens de retenue actionnables. Par ailleurs, un moyen de retenue par friction diffère d'une butée. Ainsi par exemple, des moyens de retenue par friction de la tige de poussée retiennent la tige de poussée uniquement par frottement des moyens de retenue sur la tige de poussée. En d'autres termes, les moyens de retenue par friction exercent uniquement des forces de frottement sur la tige de poussée, s'opposant au déplacement de la tige de poussée relativement aux moyens de retenue par friction.

On entend de préférence par "implant", un composé pharmaceutique à l'état solide ou semi-solide, par exemple sous forme de liquide encapsulé, et/ou un composant électronique, par exemple une puce électronique pouvant être de type RFID. On entend généralement par "patient" ou "sujet" un être vivant comme par exemple un mammifère, notamment un être humain. L'utilisateur est en général une personne différente du patient, mais il est possible que l'utilisateur soit le patient lui-même.

Dans la présente description, on comprend que la direction distale désigne la direction la plus éloignée des doigts d'un utilisateur, c'est-à-dire la plus proche de la peau ou de la surface d'un patient au moment d'une injection, et la direction proximale désigne la direction opposée à la direction distale. En d'autres termes, on considère que la direction distale et le sens distal sont la direction et le sens qui vont vers « l'avant » du dispositif d'injection d'implant, autrement appelée direction d'injection. En particulier, l'extrémité distale d'une pièce correspond à l'extrémité se trouvant du côté de l'aiguille d'injection, et l'extrémité proximale correspond à l'extrémité opposée. On comprend par ailleurs que l'axe d'injection, portant la direction d'injection, correspond à l'axe du dispositif d'injection d'implant, défini par l'axe de l'aiguille d'injection.

On comprend par conséquent que la direction « aval » est une direction opposée à la direction « amont » et correspond à la direction orientée dans le sens de l'extrémité distale du dispositif d'injection d'implant, c'est-à-dire en direction du site d'injection, vers l'extrémité configurée pour se trouver en contact avec le site d'injection de l'implant. Ainsi, la direction « aval » peut également être appelée direction d'injection. On comprend que les termes « amont » et « aval » sont relatifs aux directions distale et proximale, un élément aval étant disposé plus loin dans la direction distale qu'un élément amont.

Le dispositif d'injection d'implant peut en outre comporter l'une ou plusieurs des caractéristiques suivantes, prises seules ou en combinaison.
- Les moyens de retenue comportent :
   - un bouton d'actionnement comprenant une première surface d'appui destinée à être en contact avec la tige de poussée,
   - un support d'appui comprenant une seconde surface d'appui destinée à être en contact avec la tige de poussée,
      le bouton d'actionnement étant mobile par rapport au support d'appui entre une position de retenue, dans laquelle les première et seconde surfaces d'appui exercent chacune une première force de retenue sur la tige de poussée, et une position de libération lors de l'actionnement par un utilisateur du bouton d'actionnement, dans laquelle les surfaces d'appui exercent chacune une seconde force de retenue inférieure à la première force de retenue sur la tige de poussée, les secondes forces de retenue autorisant le déplacement de la tige de poussée vers la direction distale par rapport à l'aiguille d'injection. Ainsi, l'actionnement par un utilisateur est particulièrement aisé, du fait qu'en un simple actionnement du bouton d'actionnement, on réduit la retenue exercée par les première et seconde surfaces d'appui sur la tige de poussée, et l'on permet ainsi aux moyens de poussée de déplacer la tige de poussée.
- Au moins l'une des secondes forces de retenue est nulle. En d'autres termes, dans la position de libération du bouton d'actionnement, il n'y a plus ou sensiblement plus de contact entre la première surface d'appui et la tige de poussée et/ou entre la seconde surface d'appui et la tige de poussée.
- Le support d'appui est porté par un boîtier ou bien est formé par un boîtier.
- L'aiguille d'injection est rapportée sur un boîtier du dispositif d'injection d'implant, le boîtier pouvant en outre porter le corps de réception et les moyens d'injection. Le boîtier est de préférence un boîtier extérieur, permettant la préhension du dispositif d'injection d'implant. Par exemple, l'aiguille d'injection est solidaire du boîtier. Ainsi, lors de l'utilisation du dispositif d'injection d'implant par un utilisateur, l'aiguille d'injection reste fixe par rapport au boîtier. En particulier, elle ne se rétracte ni lors de l'injection du au moins un implant, ni après l'injection du au moins un implant. Par conséquent, cela permet de simplifier la conception du dispositif d'injection d'implant.
- Les moyens de retenue comportent des moyens de rappel du bouton d'actionnement dans sa position de retenue, notamment un ressort de rappel. Ainsi, l'actionnement par l'utilisateur est plus agréable et plus précis, car il suffit à l'utilisateur de relâcher simplement son appui sur le bouton d'actionnement pour stopper le déplacement de la tige de poussée, donc pour faire une pause dans l'injection. Ceci permet par exemple de changer l'orientation de l'aiguille d'injection pour injecter un autre implant à côté d'un premier implant et non d'injecter les deux implants dans le prolongement l'un de l'autre.
- Le dispositif d'injection d'implant comprend un élément de commande de la tige de poussée, monté coulissant par rapport à un boîtier, le boitier et l'élément de commande étant pourvus de moyens de guidage en translation de l'élément de commande par rapport au boitier. Ainsi, le positionnement de la tige de poussée et le guidage de la tige de poussée lors de son déplacement sont améliorés. L'élément de commande comprend avantageusement une douille coulissante munie d'un ergot coopérant avec une rainure portée par un boîtier et/ou munie d'une rainure coopérant avec un ergot porté par un boîtier.
- Les moyens de poussée sont automatiques. Ainsi, un utilisateur n'a pas à exercer une force de poussée dans une direction distale sur la tige de poussée, ce qui permet d'améliorer la précision de l'actionnement par l'utilisateur des moyens de retenue de la tige de poussée. Par conséquent, cela entraîne l'amélioration de la précision du processus d'injection.
- Les moyens de poussée comportent un ressort de poussée, prenant appui entre un boîtier et la tige de poussée. Le ressort de poussée peut travailler en traction ou en compression. L'utilisation d'un ressort de poussée pour exercer la poussée sur la tige de poussée est particulièrement intéressante du fait que la force du ressort de poussée permet de faciliter et rendre plus précise l'injection, tout en étant contrôlée par les moyens de retenue pour laisser à l'utilisateur le contrôle de l'injection. C'est tout particulièrement avantageux pour injecter un long implant ou une pluralité d'implants. La force maximale exercée sur la tige de poussée est définie afin d'éviter d'endommager la tige de poussée et/ou un implant et afin de faciliter l'actionnement des moyens de retenue par un utilisateur. En effet, la force devant être exercée par les moyens de retenue pour retenir la tige de poussée sera d'autant plus faible que la force exercée par le ressort de poussée est faible.
- Le dispositif d'injection d'implant comporte un élément de blocage escamotable des moyens de poussée, configuré pour dans une configuration de blocage, maintenir le dispositif d'injection d'implant dans la position initiale, dans une configuration escamotée, autoriser le passage du dispositif d'injection d'implant de passer vers sa position finale. Ainsi, le transport et la manipulation du dispositif d'injection d'implant préalablement à son utilisation sont plus sûrs, le dispositif d'injection d'implant ne pouvant être actionné par inadvertance ou à cause d'une chute de celui-ci.
- Le dispositif d'injection d'implant comporte des moyens de verrouillage agencés pour bloquer la tige de poussée dans sa position finale. Ainsi, le dispositif d'injection d'implant n'est pas réutilisable, permettant de respecter les contraintes d'hygiènes relatives à un tel dispositif d'injection d'implant.
- La tige de poussée est configurée pour pousser au moins un implant au travers de l'aiguille d'injection entre une position initiale proximale et une position finale distale dans laquelle au moins un implant est injecté. Ainsi, lors de l'utilisation du dispositif d'injection d'implant par un utilisateur, la tige de poussée n'est déplacée que dans la direction distale entre la position initiale et la position finale.
- Dans sa position finale, la tige de poussée fait saillie vers la direction distale au-delà de l'extrémité de l'aiguille d'injection. Ainsi, la tige de poussée forme dans ce cas un élément de sécurité pour l'extrémité de l'aiguille d'injection, laquelle peut être biseautée pour faciliter le piquage. La tige de poussée permet alors d'empêcher l'aiguille d'exercer sa fonction de piquage, par exemple sur une personne ou un objet en cas de chute du dispositif d'injection d'implant. En effet, dans le cas d'une chute ou d'un appui dans la direction distale, le contact avec le dispositif d'injection d'implant est ainsi réalisé par l'intermédiaire de la tige de poussée et non avec l'extrémité de l'aiguille d'injection.
- Les moyens de retenue comportent un patin porté par un boîtier, comprenant une troisième surface d'appui destinée à être en contact avec la tige de poussée pour créer une force de retenue supplémentaire sur la tige de poussée. Ainsi, la répartition des efforts radiaux exercés sur la tige de poussée est améliorée, ce qui évite sa déformation et facilite l'actionnement par l'utilisateur.
- Le bouton d'actionnement peut être un bouton d'appui ou un bouton d'actionnement latéral, lequel étant monté pivotant autour d'un axe sensiblement orthogonal à l'axe d'injection. Ces types d'actionnement sont particulièrement simples d'utilisation.
- Les moyens de retenue comprennent un patin, des moyens de rappel étant disposés entre le bouton d'actionnement et le patin. Notamment, cela facilite l'actionnement, qui est plus agréable pour un utilisateur.
- Les moyens de rappel du bouton d'actionnement comprennent un ressort de rappel travaillant en traction ou en compression.
- Les moyens de retenue sont réglables continûment, de façon à pouvoir varier manuellement la vitesse de déplacement de la tige de poussée sous l'action des moyens de poussée. Ainsi, non seulement on propose de retenir le déplacement de la tige de poussée, mais en outre on propose de pouvoir régler cette retenue continûment, de façon que la force de retenue varie graduellement, sans à-coups et pour adapter l'intensité de la retenue aux besoins, à la différence de moyens de retenue qui ne seraient configurés que pour faire une retenue complète ou pas de retenue du tout. En d'autres termes, les moyens de retenue sont actionnables par un utilisateur, quelle que soit la position de la tige de poussée entre sa position initiale et sa position finale.
- Le dispositif d'injection d'implant comprend un boîtier portant la seconde surface d'appui, la seconde surface d'appui étant en particulier venue de matière avec le boîtier.
- Le ressort de poussée est disposé entre le boîtier et la douille coulissante.
- Les moyens de verrouillage comportent une patte portée par une douille coulissante, coopérant avec un évidement porté par un boîtier.
- L'actionnement des moyens de retenue est réalisé par l'intermédiaire d'une rampe prévue sur le bouton d'actionnement ou sur le boîtier. Ainsi, la rampe facilite la maîtrise graduelle de la force de retenue qui permet un contact permanent plus ou moins marqué en fonction de l'appui de l'utilisateur sur le bouton d'actionnement.

L'invention sera mieux comprise à la lecture de la description qui va suivre, donnée uniquement à titre d'exemple et faite en se référant aux dessins dans lesquels :
- la figure 1 est une vue en perspective d'un dispositif d'injection d'implant selon un mode de réalisation, en configuration de stockage avant injection ;
- la figure 2 est une vue de côté et en perspective d'une partie du dispositif d'injection d'implant de la figure 1, dans lequel la tige de poussée est dans une position initiale ;

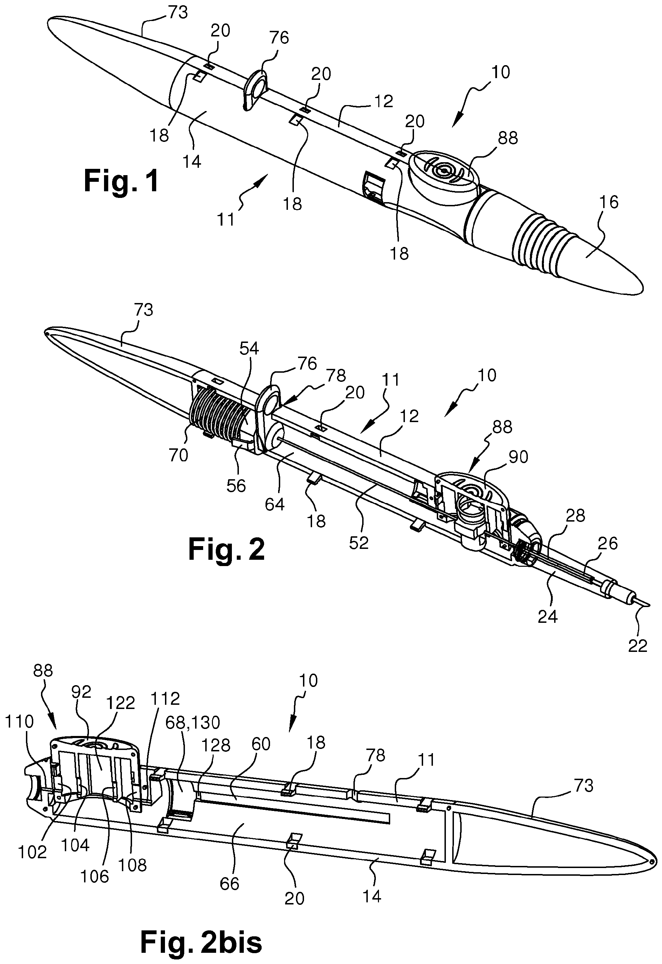

- la figure 2 bis est une vue de côté et en perspective d'une partie complémentaire du dispositif d'injection d'implant non représentée sur la figure 2 et illustré avec la partie distale orientée vers la gauche ;

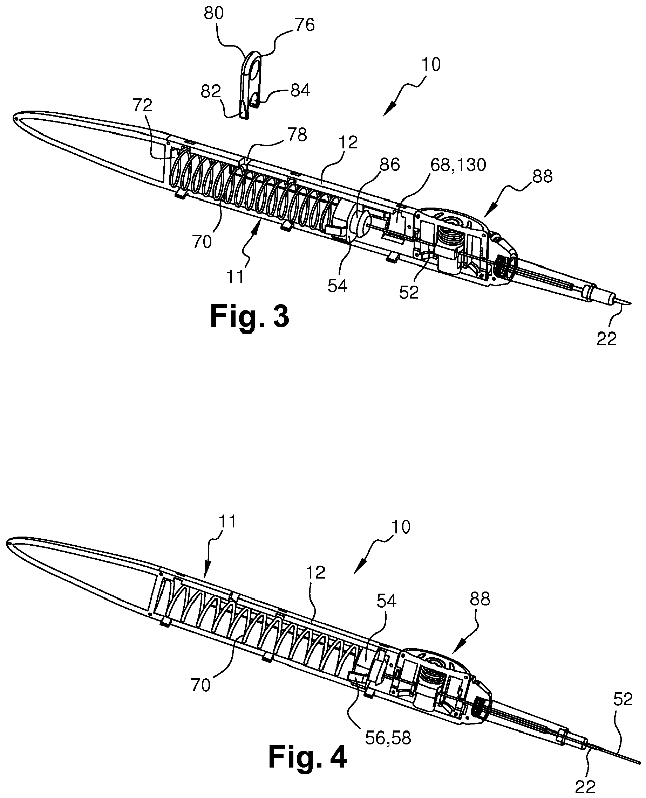

- la figure 3 est une vue similaire à la figure 2, l'élément de blocage escamotable étant escamoté, la tige de poussée étant dans une position intermédiaire ;
- la figure 4 est une vue similaire à la figure 2, la tige de poussée étant dans une position finale ;

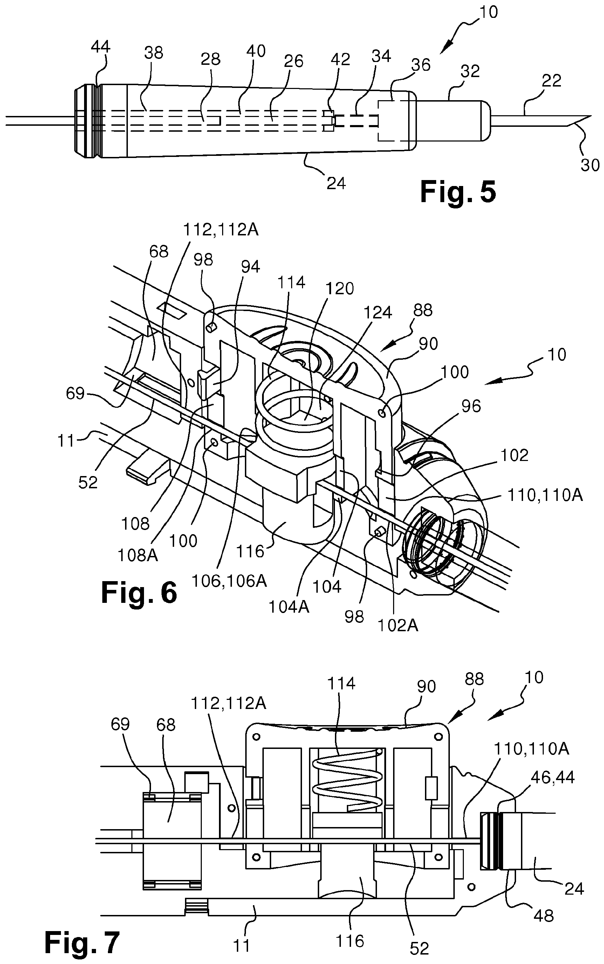

- la figure 5 est une vue du corps de réception et de l'aiguille d'injection du dispositif d'injection d'implant de la figure 2;
- la figure 6 est une vue agrandie d'une partie de la figure 2 ;
- la figure 7 est une vue de côté d'une partie du dispositif d'injection d'implant illustrée sur la figure 6 ;

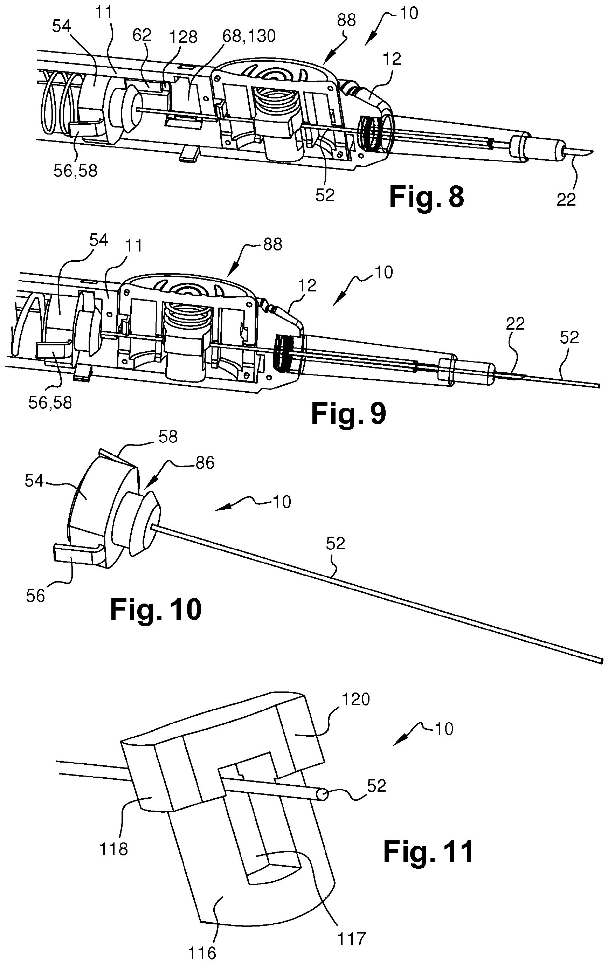

- la figure 8 est une vue agrandie d'une partie de la figure 3 ;
- la figure 9 est une vue agrandie d'une partie de la figure 4 ;
- la figure 10 est une vue en perspective d'un détail du dispositif d'injection d'implant de la figure 1 ;
- la figure 11 est une vue en perspective d'un autre détail du dispositif d'injection d'implant de la figure 1 ;

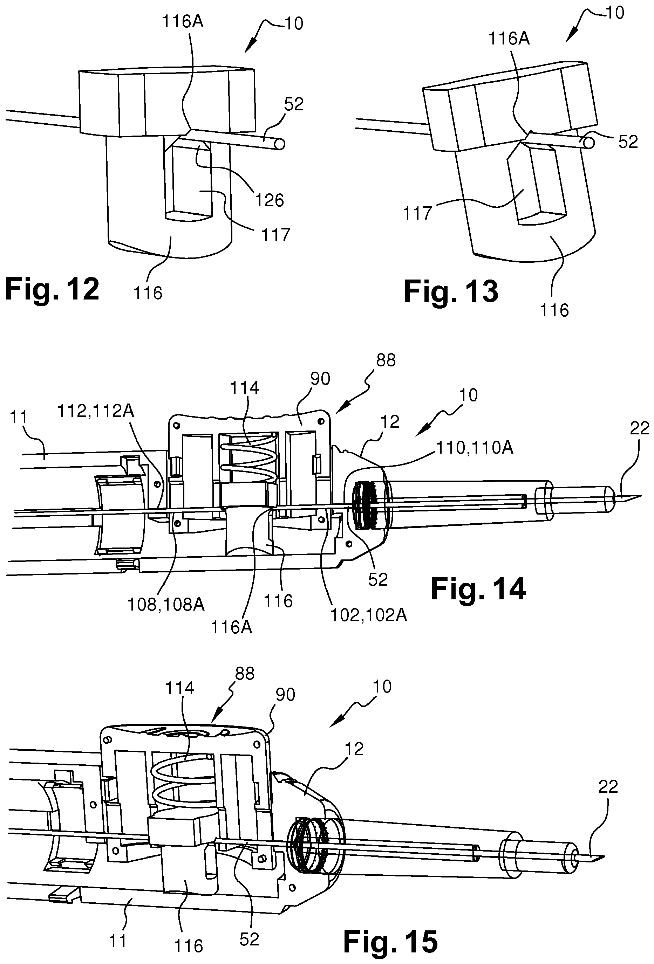

- la figure 12 est une vue en perspective d'un détail d'un dispositif d'injection d'implant selon un second mode de réalisation ;
- la figure 13 est une vue en perspective d'un détail d'un dispositif d'injection d'implant selon un troisième mode de réalisation ;
- la figure 14 est une vue en coupe et en perspective d'une partie du dispositif d'injection d'implant de la figure 12 ;
- la figure 15 est une vue en perspective d'une partie du dispositif d'injection d'implant de la figure 13.

Comme illustré sur les figures, un dispositif d'injection d'implant 10 est configuré pour injecter un ou plusieurs implants dans le corps d'un patient.

Comme illustré sur la figure 1, le dispositif d'injection d'implant 10 comporte un boîtier 11. Le boîtier 11 est ici un boîtier externe de préhension, portant une surface de préhension du dispositif d'injection d'implant 10 par un utilisateur. Il est composé dans cet exemple de deux demi-boîtiers 12, 14. Le boîtier 11 porte par ailleurs un capuchon 16 amovible.

Chaque demi-boîtier 12, 14 comporte des moyens d'assemblage, par exemple des pattes 18 et des logements 20 destinés à recevoir, à l'état assemblé par encliquetage, les pattes 18 de l'autre demi-boîtier 14, 12.

Le capuchon 16 est un capuchon de protection d'aiguille, il est ici assemblé sur le boîtier 11 par encliquetage, ou par tout autre moyen adapté, par exemple par vissage. Le capuchon 16 est dans cet exemple en forme d'ogive, munie localement de reliefs de manière à faciliter sa préhension. A l'état assemblé sur le boîtier 11, le capuchon 16 entoure une aiguille d'injection 22 (visible sur la figure 2) reliée à un corps de réception 24 (visible sur la figure 2) d'au moins un implant. De préférence, le dispositif d'injection d'implant 10 illustré est configuré pour injecter deux implants dans le corps d'un patient, notamment deux implants 26, 28 (visibles sur la figure 2). Le capuchon 16 est amovible, de manière à libérer l'aiguille d'injection 22. Ainsi, l'aiguille d'injection 22 peut, une fois le capuchon 16 ôté, être insérée dans le corps d'un patient pour permettre l'injection des implants 26, 28 dans le corps du patient.

L'aiguille d'injection 22 est creuse et est par exemple réalisée en métal comme de l'acier inoxydable. Comme illustré sur les figures et en particulier sur la figure 5, l'aiguille d'injection 22 peut comporter une extrémité 30 distale biseautée pour faciliter son insertion dans le corps du patient. L'aiguille d'injection 22 peut en outre être portée par un élément support 32, lequel peut être réalisé en matériau plastique et est destiné à limiter la profondeur d'insertion de l'aiguille d'injection 22 dans le corps du patient.

Le corps de réception 24 est rapporté ici sur le boîtier 11. Selon une variante il pourrait être réalisé directement dans le boîtier 11, en étant venu de matière avec ce dernier. Dans cet exemple, le corps de réception 24 est réalisé en un matériau plastique transparent. Ainsi, un utilisateur est en mesure de détecter visuellement la présence des implants 26, 28 dans le dispositif d'injection d'implant 10 avant de réaliser l'injection, lorsque le capuchon 16 est ôté. Alternativement, une lumière pourrait être ménagée dans le corps de réception 24.

Le corps de réception 24 est disposé en amont de l'aiguille d'injection 22, et est destiné à recevoir les implants 26, 28, de sorte que les implants 26, 28 soient disposés en amont de l'aiguille d'injection 22, dans la direction d'injection de l'aiguille d'injection 22.

Le corps de réception 24 se présente par exemple sous la forme d'un cylindre, de forme légèrement tronconique. Ainsi comme on peut le voir sur la figure 5, le corps de réception 24 est creux et comporte à son extrémité distale un tronçon 34 doté d'un diamètre interne correspondant sensiblement au diamètre externe de l'aiguille d'injection 22. Ainsi, l'aiguille d'injection 22 peut être insérée dans le tronçon 34. Le corps de réception 24 peut en outre comporter un alésage 36 destiné à loger une partie de l'élément support 32. Cela permet de rigidifier l'ensemble formé par le corps de réception 24, l'aiguille d'injection 22 et l'élément support 32.

Le corps de réception 24 peut également comporter un alésage 38 destiné à loger un organe de réception 40, lequel peut contenir les implants 26, 28. L'organe de réception 40 peut être formé par un tube, notamment en matériau plastique transparent. Ainsi, il peut contenir les implants 26, 28. Par exemple, dans le cas où l'organe de réception 40 contient deux implants 26, 28, les deux implants 26, 28 sont disposés l'un derrière l'autre, c'est-à-dire l'un en amont de l'autre, selon la direction d'injection. L'organe de réception 40 peut comporter une extrémité distale 42 dotée d'un moyen de retenue d'implant, comme une membrane ou un faible rétrécissement de son diamètre interne, ayant pour but d'éviter la chute d'un implant à travers l'aiguille d'injection 22, par exemple sous l'action de la force de gravité terrestre. L'extrémité distale 42 peut alternativement comporter un élément souple de retenue de l'implant 26, 28 comportant un orifice d'un diamètre inférieur à celui de l'implant 26, 28, lequel est configuré pour se déformer et permettre le passage de l'implant 26, 28 vers l'aiguille d'injection 22 lors de l'injection.

Comme illustré sur la figure 7, le corps de réception 24 comporte enfin dans cet exemple une extrémité proximale comportant un élément d'encliquetage, comme par exemple une rainure périphérique 44. De cette manière, le corps de réception 24 est fixé sur le boîtier 11, le boîtier 11 comportant un élément d'encliquetage complémentaire, comme par exemple une protubérance périphérique 46 formée dans un alésage 48, la protubérance périphérique 46 coopérant avec la rainure d'encliquetage périphérique 44, visible sur la figure 7.

Pour permettre l'injection des implants 26, 28, le dispositif d'injection d'implant 10 comporte des moyens d'injection. Ces moyens d'injection sont ici disposés dans le boîtier 11.

Comme illustré sur la figure 2, les moyens d'injection comportent une tige de poussée 52, des moyens de poussée sur la tige de poussée 52 et des moyens de retenue par friction de la tige de poussée 52.

La tige de poussée 52 peut être réalisée en métal, par exemple en acier, de préférence en acier inoxydable La tige de poussée 52 est disposée en amont d'un implant, par exemple en amont de l'implant 28 qui est lui-même l'implant disposé le plus en amont parmi les implants 26, 28. La tige de poussée 52 est ainsi configurée pour pousser l'implant, par exemple les implants 26, 28, au travers de l'aiguille d'injection 22 entre une position initiale et une position finale. Ainsi, dans la position initiale de la tige de poussée 52, les implants 26, 28 sont logés dans le corps de réception 24, et dans la position finale de la tige de poussée 52, les implants 26, 28 sont passés à travers l'aiguille d'injection 22 et se trouvent a priori placés dans le corps d'un patient.

Le dispositif d'injection d'implant 10 comporte en outre ici un élément de commande 54 de la tige de poussée 52, monté coulissant par rapport au boîtier 11. L'élément de commande 54 comprend une douille coulissante munie d'un ergot coopérant avec une rainure portée par le boîtier 11 et/ou munie d'une rainure coopérant avec un ergot porté par le boîtier 11. Plus précisément, l'élément de commande 54 est solidaire de la tige de poussée 52 et est formé par la douille coulissante, comme illustré en particulier sur la figure 10. L'élément de commande 54 et le boîtier 11 comportent des moyens de guidage en translation de l'élément de commande 54 par rapport au boîtier 11. Les moyens de guidage en translation comportent par exemple un ergot, ou bien une patte 56, 58, porté par l'élément de commande 54, et une rainure 60, 62 (visible sur la figure 2 bis) en regard de l'ergot ou de la patte 56, 58 et pratiquée sur une paroi interne du boîtier 11, de manière à coopérer avec l'ergot ou la patte 56, 58. En particulier, l'élément de commande 54 comporte deux pattes 56, 58, chacune en regard d'une rainure 60, 62 pratiquée sur une paroi interne 64, 66 d'un demi-boîtier 12, 14 respectif.

Selon un autre mode de réalisation non représenté, le boîtier peut comporter une nervure, l'élément de commande une rainure apte à recevoir la nervure, la nervure et la rainure faisant office de d'ergot et de rainure dont les éléments qui les portent sont inversés.

Les moyens de guidage en translation sont conformés pour guider l'élément de commande 54 et la tige de poussée 52 en translation selon la direction d'injection de l'aiguille d'injection 22.

Le boîtier 11 peut comporter une ouverture, en particulier une ouverture 130 sur chaque demi-boîtier 12, 14. Chaque ouverture 130 peut être fermée par un capot 68 visible sur la figure 2bis, lequel peut être fixé par encliquetage sur le boîtier 11, en particulier sur chaque demi-boîtier 12, 14. Pour cela, le capot 68 comporte une pluralité de pattes 69, visibles sur la figure 6, en particulier quatre pattes 69, lesquelles coopèrent avec des rainures pour la fixation du capot 68 sur le boîtier 11, en particulier sur chaque demi-boîtier 12, 14. Par exemple, le capot 68 est réalisé en matériau plastique transparent. Le capot 68 peut avantageusement être placé au niveau de l'élément de commande 54, lorsque la tige de poussée 52 occupe une position finale. Ainsi, un utilisateur peut visuellement détecter que le dispositif d'injection d'implant 10 a atteint une position finale d'injection. Le capot 68 peut également avoir une forme correspondant à celle du boîtier 11, en particulier il peut affleurer extérieurement le boîtier 11 lorsqu'il est monté sur le boîtier 11.

Le boîtier 11 comporte en outre une fenêtre traversante 132 recevant un bouton d'actionnement décrit plus loin.

Les moyens de poussée sur la tige de poussée 52 comportent dans cet exemple un ressort de poussée 70 (visible sur les figures 3 et 4), lequel prend appui entre le boîtier 11 et la tige de poussée 52. En particulier, le ressort de poussée 70 est un ressort travaillant en compression. Alternativement, le ressort de poussée peut être un ressort travaillant en traction. Par exemple, le ressort de poussée 70 prend appui côté boîtier 11 sur une butée interne 72 formée par une surface sensiblement plane sur le boîtier 11. Comme illustré en particulier sur la figure 3, la butée interne 72 peut être formée partiellement sur chaque demi-boîtier 12, 14. Le ressort de poussée 70 prend par ailleurs appui côté tige de poussée 52 sur une butée formée par une surface sensiblement plane sur l'élément de commande 54, cette butée se trouvant en regard de la butée interne 72 formée sur le boîtier 11. En particulier, le ressort de poussée 70 est disposé entre le boîtier 11 et la douille coulissante 54.

Comme on peut le voir notamment sur la figure 2, le boîtier 11 est, à son extrémité 73 opposée à la direction d'injection, en forme d'ogive pour faciliter sa préhension. En effet, un avantage du dispositif d'injection d'implant 10 est qu'il est utilisable d'une seule main. En particulier, le boîtier 11 peut être formé en forme d'ogive au-delà de la butée interne 72, dans la direction opposée à la direction d'injection.

La force fournie par le ressort de poussée 70 est transmise à la tige de poussée 52 par l'intermédiaire de l'élément de commande 54. La tige de poussée 52 peut alors exercer une force permettant l'injection d'un implant, en particulier des implants 26, 28, dans le corps d'un patient. Ainsi, le ressort de poussée 70 est configuré pour pousser la tige de poussée 52 depuis sa position initiale jusqu'à sa position finale.

Le dispositif d'injection d'implant 10 peut en outre comporter un élément de blocage escamotable 76 des moyens de poussée, visible notamment sur les figures 2 et 3. L'élément de blocage escamotable 76 permet, lorsqu'il est en configuration de blocage, d'empêcher que les moyens de poussée provoquent par accident le déplacement de la tige de poussée 52, notamment lors du transport ou du stockage du dispositif d'injection d'implant 10. Par exemple, l'élément de blocage escamotable est formé par une clé 76. L'élément de blocage escamotable 76 autorise, lorsqu'il est en configuration escamotée, le déplacement de la tige de poussée 52.

Dans la position initiale de la tige de poussée, comme illustrée sur la figure 2, la clé 76 est engagée dans un orifice 78 ménagé dans le boîtier 11, en particulier de manière complémentaire sur chaque demi-boîtier 12, 14. Comme illustré sur la figure 3, la clé 76 est formée à une de ses extrémités par une extrémité de préhension 80. L'autre extrémité est par exemple formée par deux branches 82, 84, lesquelles sont configurées pour s'engager dans une rainure périphérique 86 (visible sur la figure 10) ménagée sur l'élément de commande 54, en particulier sur la douille coulissante. De cette manière, un déplacement de l'élément de commande 54 dans la direction d'injection est empêché, les efforts étant transmis depuis les moyens de poussée à l'élément de commande 54, puis de l'élément de commande 54 à la clé 76, et de la clé 76 au boîtier 11 au niveau des parois de l'orifice 78. Ainsi, la clé 76 doit être retirée avant d'utiliser le dispositif d'injection d'implant 10 pour injecter un implant 26, 28 dans le corps d'un patient.

Les moyens de retenue par friction de la tige de poussée 52 sont configurés pour retenir la tige de poussée 52 par rapport à l'aiguille d'injection 22 et au boîtier 11. Ces moyens de retenue s'opposent au déplacement de la tige de poussée 52 vers sa position finale et donc à l'injection des implants 26, 28. Ces moyens de retenue sont actionnables par un utilisateur.

Les moyens de retenue comportent par exemple un bouton d'actionnement 88.

Le bouton d'actionnement 88 fait partiellement saillie hors du boîtier 11 par la fenêtre traversante 132 pour permettre l'actionnement par un utilisateur, notamment par appui. Il peut être composé de deux demi-boutons 90, 92, visibles sur les figues 2 et 2bis. Chaque demi-bouton 90, 92 comporte des moyens d'assemblage, à savoir ici une patte 94 et un logement 96 destiné à la recevoir (visibles sur la figure 6), à l'état assemblé par encliquetage, la patte 94 de l'autre demi-bouton 92, 90. Chaque demi-bouton 90, 92 peut en outre comporter des pions de positionnement 98 et des trous de positionnement 100 destinés à recevoir (également visibles sur la figure 6),, à l'état assemblé du bouton d'actionnement 88, les pions de positionnement de l'autre demi-bouton 92, 90.

Dans l'exemple illustré notamment sur les figures 1 et 2, 6 et 7, le bouton d'actionnement 88 est traversé par la tige de poussée 52, laquelle passe au travers de trous formés par des rainures respectives 102, 104, 106, 108 ménagées sur chaque demi-bouton 90, 92, disposées en regard de manière à former des trous lorsqu'elles sont prises deux à deux, ces trous étant alignés axialement entre eux. Bien que plusieurs trous soient ainsi formés sur l'exemple illustré, en particulier deux ou quatre trous, un trou unique peut être formé dans le bouton d'actionnement, le trou étant traversé par la tige de poussée.

Le bouton d'actionnement 88 comporte une première surface d'appui destinée à être en contact avec la tige de poussée 52, en particulier lorsque les moyens de retenue ne sont pas actionnés par l'utilisateur. La surface d'appui est formée par la paroi d'un des trous formés par les rainures 102, 104, 106, 108. Par exemple, le bouton d'actionnement 88 peut comporter quatre premières surfaces d'appui 102A, 104A, 106A, 108A situées sur un bord des rainures 102, 104, 106, 108. Ce bord de chacune de ces rainures 102, 104, 106, 108 peut être le bord inférieur, lequel est opposé au bord supérieur de ces rainures 102, 104, 106, 108 qui se trouve plus proche de la partie du bouton d'actionnement 88 faisant saillie hors du boîtier 11 que le bord inférieur.

Les moyens de retenue comportent en outre un support d'appui 110, 112, 116, visible par exemple sur les figures 6 et 14. Le support d'appui 110, 112, 116 comporte une seconde surface d'appui 110A, 112A destinée à être en contact avec la tige de poussée 52, en particulier lorsque les moyens de retenue ne sont pas actionnés par l'utilisateur. Dans l'exemple illustré, le support d'appui est au moins partiellement formé par des parties 110, 112 du boîtier 11. En d'autres termes, la seconde surface d'appui 110A, 112A comporte une surface portée par le boîtier 11. En particulier, la seconde surface d'appui 110A, 112A comporte des surfaces internes de trous de guidage 110, 112 de la tige de poussée 52, les trous de guidage 110, 112 étant formés dans le boîtier 11. Ainsi dans ce cas, la seconde surface d'appui 110A, 112A est formée par le boîtier 11, la seconde surface d'appui 110A, 112A étant venue de manière avec le boîtier 11. Le support d'appui 110, 112, 116 peut éventuellement comprendre, par ailleurs, un patin 116, comme cela est décrit dans la suite.

Le bouton d'actionnement 88 est mobile par rapport au support d'appui 110, 112, 116 entre une position de retenue et une position de libération lors de l'actionnement par un utilisateur du bouton d'actionnement 88. Dans la position de retenue, les premières et secondes surfaces d'appui 102A, 104A, 106A, 108A, 110A, 112A exercent chacune une première force de retenue sur la tige de poussée 52. Dans la position de libération, les premières et secondes surfaces d'appui 102A, 104A, 106A, 108A, 110A, 112A exercent chacune une seconde force de retenue inférieure à la première force de retenue sur la tige de poussée 52. Les secondes forces de retenue autorisent alors le déplacement de la tige de poussée 52 vers la direction distale par rapport à l'aiguille d'injection 22, c'est-à-dire dans la direction d'injection. Au moins l'une des secondes forces de retenue peut être nulle. Dans ce cas, il peut ne plus y avoir de contact entre la tige de poussée 52 et l'une des premières et secondes surfaces d'appui 102A, 104A, 106A, 108A, 110A, 112A.

Les moyens de retenue comportent des moyens de rappel du bouton d'actionnement 88. Les moyens de rappel du bouton d'actionnement 88 peuvent comporter un ressort, comme un ressort de rappel 114 travaillant par exemple en compression. Alternativement, le ressort de rappel peut travailler en traction.

Les moyens de retenue comportent par ailleurs un patin 116, les moyens de rappel 114 étant disposés entre le bouton d'actionnement 88 et le patin 116.

Le patin 116 est rapporté ici sur le boîtier 11 et comporte un trou 117 pour le passage de la tige de poussée 52 (visible sur la figure 11). Il comporte en outre des protubérances 118, 120 visibles sur la figure 11 et coopérant avec des rainures 122, 124 ménagées dans le bouton d'actionnement 88 et visibles respectivement sur les figures 2bis et 6. Ainsi dans ce cas, le bouton d'actionnement 88 ne peut se déplacer qu'en translation le long des rainures 122, 124 selon une direction sensiblement orthogonale à la direction d'injection, sur une course limitée par des butées inférieures et supérieures des rainures 122, 124.

Dans l'exemple illustré, le bouton d'actionnement 88 est monté coulissant dans le boîtier 11, selon une direction sensiblement orthogonale à la direction d'injection. Alternativement, le bouton d'actionnement 88 peut être un bouton d'actionnement latéral, monté pivotant autour d'un axe sensiblement orthogonal à l'axe d'injection.

Enfin, les moyens de retenue peuvent comporter une rampe d'appui formée sur le bouton d'actionnement 88, le boîtier 11 ou le patin 116. Ainsi, lorsque l'utilisateur appuie sur le bouton d'actionnement 88, la force de retenue exercée par la rampe d'appui sur la tige de poussée 52 diminue, ce qui permet le déplacement de la tige de poussée 52. Par rampe d'appui, on peut notamment comprendre une surface sensiblement plane, inclinée par rapport au plan défini par la direction d'injection et la direction de la force de rappel exercée par le ressort de rappel 114, dont l'intersection avec ce plan définit un axe sensiblement parallèle à la direction d'injection.

Dans l'exemple illustré, les moyens de retenue sont réglables continûment. Ainsi, la vitesse de déplacement de la tige de poussée 52 sous l'action des moyens de poussée, en particulier le ressort de poussée 70, peut varier manuellement. En effet, grâce au bouton d'actionnement 88, un utilisateur peut actionner celui-ci par exemple par appui avec une force variable. Si la force d'appui est plus importante, les secondes forces de retenue sont plus faibles et la vitesse de déplacement de la tige de poussée 52 augmente. En particulier, au moins une force de retenue peut être nulle. Au contraire, si la force d'appui est moins importante, les frottements sont plus importants, de sorte que les secondes forces de retenue sont plus élevées et la vitesse de déplacement de la tige de poussée 52 diminue.

Les moyens de retenue comportent un patin, porté par le boîtier 11 comme illustré sur les figures 12 à 15. Le patin peut être formé par le patin 116. Il comprend une troisième surface d'appui 116A, destinée à être en contact avec la tige de poussée 52 pour créer une force de retenue supplémentaire sur la tige de poussée 52, en particulier lorsque les moyens de retenue ne sont pas actionnés par l'utilisateur. Alternativement, le patin 116 peut former le support d'appui 110, 112 comprenant la troisième surface d'appui 116A destinée à être en contact avec la tige de poussée 52. Dans ce cas, les trous de guidage 110, 112 peuvent ne pas former la seconde surface d'appui 110A, 112A, la seconde surface d'appui étant alors formée par la troisième surface d'appui 116A.

Dans l'exemple illustré sur les figures 12 et 14, le patin 116 comporte un trou 117 de passage de la tige de poussée 52 de forme particulière. La partie supérieure du trou 117 possède une forme complémentaire de celle de la tige de poussée 52, c'est-à-dire partiellement cylindrique, de manière à augmenter la surface de contact entre la tige de poussée 52 et la paroi de la partie supérieure du trou 117 pour former la troisième surface d'appui 116A. Ainsi, la force de retenue supplémentaire peut être augmentée, du fait des forces de frottements présentes au niveau de la troisième surface d'appui 116A. Le trou 117 peut également comporter des parois de guidage 126 de la tige de poussée 52 vers la troisième surface d'appui 116A, par exemple en forme de V.

L'exemple de patin 116 illustré sur les figures 13 et 15 se distingue de celui illustré sur les figures 12 et 14 en ce que la partie supérieure du trou 117 est formée par le prolongement des parois de guidage 126 en forme de V. Ainsi, la troisième surface d'appui 116A entre la tige de poussée 52 et la paroi de la partie supérieure du trou 117 est formée sensiblement par deux zones de contact longitudinales rectilignes.

Au moins l'une parmi la première surface d'appui 102A, 104A, 106A, 108A, la seconde surface d'appui 110A, 112A et le cas échéant la troisième surface d'appui 116A possède un coefficient de frottement statique élevé avec la tige de poussée 52, par exemple supérieur à 0,5. En particulier, la première surface d'appui 102A, 104A, 106A, 108A et/ou la troisième surface d'appui 116A comporte un coefficient de frottement statique élevé avec la tige de poussée 52, par exemple supérieur à 0,5. Dans la suite on utilise le terme surface d'appui pour les premières, secondes et troisième surfaces d'appui 102A, 104A, 106A, 108A, 110A, 112A, 116A.

Ainsi, les caractéristiques de frottement entre deux surfaces sont couramment décrites par deux coefficients de frottement : statique µₛ et cinétique µₖ.

Les principaux paramètres qui interviennent sont :
- la force exercée par la surface d'appui considérée sur la tige de poussée 52, fonction de la pression de contact. La pression de contact dépend notamment de la longueur du contact entre la surface d'appui et la tige de poussée 52, par exemple supérieure ou égale à 25 mm, et du diamètre de la tige de poussée 52, par exemple inférieur ou égal à 1 mm ;
- les matériaux de chaque surface en contact : en considérant que la tige de poussée 52 est en acier, et que le matériau d'au moins une surface d'appui, par exemple le matériau du patin 116, est un matériau plastique frottant, les surfaces en contact peuvent présenter un coefficient de frottement statique élevé, typiquement au-dessus de 0,5.

Un matériau plastique frottant peut inclure au moins l'un des matériaux choisis parmi la liste suivante : les polymères non chargés présentant un taux de cristallinité faible, et présentant généralement une température de transition vitreuse inférieure à la température ambiante ; les thermoplastiques élastomères, comme le TPE, le TPU ; les thermodurcissables tels que les gommes naturelles, possédant par exemple une température de transition vitreuse de -70°C ; le polyuréthane ; les silicones. En outre, l'ajout de nodules en caoutchouc et/ou d'autres charges dans un matériau polymère permet également d'augmenter le coefficient de frottement, ce qui permet d'élargir le choix du matériau polymère formant base.

Le ressort de poussée 70 et le ressort de rappel 114 peuvent être réalisés en métal, par exemple en acier inoxydable.

Les autres éléments du dispositif d'injection d'implant 10 dont le matériau n'est pas précisé dans la présente description peuvent être réalisés en matériau thermoplastique, par exemple en polyéthylène ou en polypropylène.

Un exemple de fonctionnement du dispositif d'injection d'implant 10 va à présent être décrit.

Le dispositif d'injection d'implant 10 comme illustré sur la figure 1 se trouve en configuration de stockage, avant utilisation.

L'utilisateur doit retirer le capuchon 16, comme illustré sur la figure 2 - le dispositif d'injection d'implant 10 étant considéré assemblé - il vérifie la présence de l'implant ou des implants 26, 28 par visualisation à travers le corps de réception 24.

L'utilisateur peut ensuite retirer la clé 76. A ce stade, la tige de poussée 52 est soumise à l'action du ressort de poussée 70. Cependant, la tige de poussée 52 est immobile et n'est pas déplacée sous l'action du ressort de poussée 70, du fait des moyens de retenue par friction, lesquels exercent des premières forces de retenue sur la tige de poussée 52. En particulier, la tige de poussée 52 est retenue par les forces de retenue exercées par les premières surfaces d'appui 102A, 104A, 106A, 108A formées par les rainures 102, 104, 106, 108, et par les secondes surface d'appui 110A, 112A formées par les trous de guidage 110, 112 et/ou par un patin 116.

Comme illustré sur les figures 3 et 8, la clé 76 étant retirée, l'aiguille d'injection 22 est ensuite insérée dans le corps du patient, et l'utilisateur appuie sur le bouton d'actionnement 88 pour libérer le déplacement de la tige de poussée 52. Cela provoque le déplacement de la tige de poussée 52 sous l'action du ressort de poussée 70, laquelle pousse un implant, en particulier le premier implant 26, dans le corps du patient. Ainsi sur la figure 3, la tige de poussée est représentée en position intermédiaire, ce qui correspond dans l'exemple illustré à l'injection réalisée d'un premier implant 26 dans le corps du patient, un second implant 28 restant dans l'aiguille d'injection 22. Comme cela a été décrit précédemment, il est possible de faire varier la vitesse de déplacement de la tige de poussée 52, voire de stopper le déplacement de la tige de poussée 52 sous l'action des moyens de retenue par friction. Cela peut être souhaité par exemple entre chaque injection d'implant, dans le cas où le dispositif d'injection d'implant 10 est utilisé pour l'injection de plusieurs implants 26, 28, car cela permet de modifier l'orientation de l'aiguille d'injection 22 donc la direction d'injection par rapport à la peau pour que les deux implants 26, 28 soient injectés en parallèle selon deux orientations distinctes, et non en série, afin de limiter la profondeur d'injection des implants 26, 28. Cela peut également être souhaité dans le cas où l'utilisateur souhaite déplacer le dispositif d'injection d'implant 10 après l'injection d'un premier implant 26 pour partir d'un autre point d'injection pour injecter un second implant 28.

Les figures 4 et 9 illustrent le dispositif d'injection d'implant 10 dans la position finale de la tige de poussée 52. Dans cette position finale, la tige de poussée 52 fait saillie vers la direction distale au-delà de l'extrémité 30 de l'aiguille d'injection 22. Toujours dans cette position finale, l'élément de commande 54 se trouve en butée contre une surface de butée du boîtier 11. De plus, le dispositif d'injection d'implant 10 comporte des moyens de verrouillage agencés pour bloquer la tige de poussée 52 dans la position finale. Ainsi dans cette position finale, l'ergot 56, 58 de l'élément de commande 54 coopère avec un évidement 128 porté par le boîtier 11, en particulier ménagé dans le boîtier 11 à l'extrémité de la rainure 60, 62 vers la direction distale, lequel est visible sur la figure 8. Avantageusement, le passage de l'ergot 56, 58 de la rainure 60, 62 à l'évidement 128 génère un signal sonore audible par l'utilisateur, comme un 'clic'.

La course de la tige de poussée 52 entre la position initiale et la position finale peut par exemple s'étendre sur une longueur de 10 à 50 mm, en particulier entre 30 et 40 mm, de préférence configurée pour l'injection de deux implants 26, 28 ayant chacun une longueur voisine de 8 mm.

Un exemple d'assemblage du dispositif d'injection d'implant 10 va à présent être décrit.

Tout d'abord, un capot 68 est monté dans chaque demi-boîtier 12, 14 par encliquetage des pattes 69 sur le demi-boîtier 12, 14 respectif. Le ressort de poussée 70 est ensuite disposé dans le demi-boîtier 12 contre la butée interne 72. Le ressort de poussée 70 est comprimé et l'élément de commande 54 est disposé dans le demi-boîtier 12, contre le ressort de poussée 70. L'élément de commande 54 est ensuite déplacé dans une position permettant l'insertion de la clé 76 dans l'orifice 78 et le maintien en position de l'élément de commande 54 par la clé 76 par l'intermédiaire de l'engagement des branches 82, 84 de la clé 76 dans la rainure périphérique 86 de l'élément de commande 54. Ensuite, le demi-bouton 90 et le patin 116 sont disposés dans le demi-boîtier 12. Le ressort de rappel 114 est alors placé entre le demi-bouton 90 et le patin 116. Après cela, la tige de poussée est insérée à travers le trou 117 du patin 116 de bouton d'actionnement 88 et est engagée dans l'élément de commande 54 de manière à être solidaire de l'élément de commande 54. Le demi-bouton 92 est ensuite rapporté sur le demi-bouton 90 de manière à former le bouton d'actionnement 88. Le demi-boîtier 14 équipé du capot 68 est alors rapporté sur le demi-boîtier 12 équipé des éléments précités.

Indépendamment de cela, l'aiguille d'injection 22 peut être équipée de l'élément support 32. En particulier, l'élément support 32 est surmoulé sur l'aiguille d'injection 22. Alternativement l'élément support 32 peut être engagé en force sur l'aiguille d'injection 22 de manière à ce que l'ajustement entre l'élément support 32 et l'aiguille d'injection soit serré. Le corps de réception 24 est ensuite monté serré ou surmoulé sur l'ensemble ainsi réalisé. L'organe de réception 40, logeant les implants 26, 28 est alors inséré dans le corps de réception 24 dans l'alésage 38.

Enfin, l'ensemble formé par l'aiguille d'injection 22, le corps de protection 24, l'élément support 32, l'organe de réception 40 et les implants 26, 28 est assemblé sur le boîtier 11 dans l'alésage 48, par encliquetage de la rainure d'encliquetage périphérique 44 du corps de protection 24 dans la protubérance périphérique 46 du boîtier 11. Il est à noter que cette dernière étape peut éventuellement être effectuée ultérieurement, par exemple par l'utilisateur.

L'invention n'est pas limitée aux modes de réalisation présentés et d'autres modes de réalisation apparaîtront clairement à l'homme du métier.

Il est notamment possible que l'utilisateur et le patient soient une même personne. Bien que le dispositif d'injection d'implant 10 soit en particulier illustré avec deux implants 26, 28, le dispositif d'injection d'implant 10 peut être configuré pour injecter un unique implant de grande longueur. Alternativement, le dispositif d'injection d'implant 10 peut être configuré pour injecter une pluralité d'implants sous forme de billes ou de sphères creuses contenant un produit, par exemple entre 2 et 50 implants, plus précisément entre 10 et 20 implants.

## Revendications

1. Dispositif d'injection d'implant (10), **caractérisé en ce qu'**il comporte :
- une aiguille d'injection (22),
- un corps de réception (24) d'au moins un implant (26, 28),
- des moyens d'injection comprenant :
. une tige de poussée (52), disposée en amont de l'implant (26, 28) logé dans le corps de réception (24) et configurée pour pousser l'implant (26, 28) au travers de l'aiguille d'injection (22) entre une position initiale et une position finale,
. des moyens de poussée sur la tige de poussée (52) entre la position initiale et la position finale,
. des moyens de retenue par friction de la tige de poussée (52) par rapport à l'aiguille d'injection (22), s'opposant au déplacement de la tige de poussée (52) vers sa position finale, les moyens de retenue étant actionnables par un utilisateur.

2. Dispositif d'injection d'implant (10) selon la revendication précédente, dans lequel les moyens de retenue comportent :
- un bouton d'actionnement (88) comprenant une première surface d'appui (102A, 104A, 106A, 108A) destinée à être en contact avec la tige de poussée (52),
- un support d'appui (110, 112, 116) comprenant une seconde surface d'appui (110A, 112A) destinée à être en contact avec la tige de poussée (52),
le bouton d'actionnement (88) étant mobile par rapport au support d'appui (110, 112, 116) entre une position de retenue, dans laquelle les première et seconde surfaces d'appui (102A, 104A, 106A, 108A, 110A, 112A) exercent chacune une première force de retenue sur la tige de poussée (52), et une position de libération lors de l'actionnement par un utilisateur du bouton d'actionnement (88), dans laquelle les première et seconde surfaces d'appui (102A, 104A, 106A, 108A, 110A, 112A) exercent chacune une seconde force de retenue inférieure à la première force de retenue sur la tige de poussée (52), les secondes forces de retenue autorisant le déplacement de la tige de poussée (52) vers la direction distale par rapport à l'aiguille d'injection (22).

3. Dispositif d'injection d'implant (10) selon la revendication précédente, dans lequel les moyens de retenue comportent des moyens de rappel du bouton d'actionnement (88) dans sa position de retenue, notamment un ressort de rappel (114).

4. Dispositif d'injection d'implant (10) selon l'une quelconque des revendications précédentes, comprenant un élément de commande (54) de la tige de poussée (52), monté coulissant par rapport à un boîtier (11), le boîtier (11) et l'élément de commande (54) étant pourvus de moyens de guidage en translation de l'élément de commande (54) par rapport au boîtier (11).

5. Dispositif d'injection d'implant (10) selon l'une quelconque des revendications précédentes, dans lequel les moyens de poussée comportent un ressort de poussée (70), prenant appui entre un boîtier (11) et la tige de poussée (52).

6. Dispositif d'injection d'implant (10) selon l'une quelconque des revendications précédentes, comportant un élément de blocage (76) escamotable des moyens de poussée, configuré pour
- dans une configuration de blocage, maintenir le dispositif d'injection d'implant (10) dans la position initiale,
- dans une configuration escamotée, autoriser le passage du dispositif d'injection d'implant (10) vers sa position finale.

7. Dispositif d'injection d'implant (10) selon l'une quelconque des revendications précédentes, comportant des moyens de verrouillage agencés pour bloquer la tige de poussée (52) dans sa position finale.

8. Dispositif d'injection d'implant (10) selon la revendication précédente, dans lequel dans sa position finale, la tige de poussée (52) fait saillie vers la direction distale au-delà de l'extrémité (30) de l'aiguille d'injection (22).

9. Dispositif d'injection d'implant (10) selon l'une quelconque des revendications précédentes, dans lequel les moyens de retenue comportent un patin (116) porté par un boîtier (11), comprenant une troisième surface d'appui (116A) destinée à être en contact avec la tige de poussée (52) pour créer une force de retenue supplémentaire sur la tige de poussée (52).
